Europäisches Patentamt

European Patent Office

Office européen des brevets

(19)

(11) Veröffentlichungsnummer: **0 036 943**
B1

(12) **EUROPÄISCHE PATENTSCHRIFT**

(45) Veröffentlichungstag der Patentschrift:
06.06.84

(21) Anmeldenummer: 81101250.9

(22) Anmeldetag: 21.02.81

(51) Int. Cl³: **B 01 J 8/00,** B 01 J 19/00,
B 65 G 53/08

(54) Verfahren zum Einführen von festem Cyanurchlorid in eine Cyanurchloridschmelze.

(30) Priorität: 27.03.80 DE 3011860

(43) Veröffentlichungstag der Anmeldung:
07.10.81 Patentblatt 81/40

(45) Bekanntmachung des Hinweises auf die Patenterteilung:
06.06.84 Patentblatt 84/23

(84) Benannte Vertragsstaaten.
BE CH DE FR GB LI

(56) Entgegenhaltungen:
DE - A - 2 165 855
DE - A - 2 617 374
DE - B - 1 127 334
US - A - 3 099 496
US - A - 3 460 869

(73) Patentinhaber: Degussa Aktiengesellschaft,
Weissfrauenstrasse 9, D-6000 Frankfurt am Main 1 (DE)

(72) Erfinder: Kriebitzsch, Norbert, Dr., Castellstrasse 2,
D-6451 Hammersbach 1 (DE)
Erfinder: Puschner, Kurt, In den Steinäckern 13,
D-6458 Rodenbach 1 (DE)
Erfinder: Hentschel, Klaus, Dr., Merellaan 9,
B-2180 Kalmthout (BE)

## Beschreibung

Bekanntlich hat die Handhabung von flüssigem Cyanurchlorid gegenüber der mit der festen Form große Vorteile, da sich die Cyanurchloridschmelze in isolierten, beheizbaren Tankzügen transportieren und in ebensolchen Tanks lagern läßt. Der Kontakt des Personals mit Cyanurchlorid ist damit entsprechend verringert worden. Außerdem ist flüssiges Cyanurchlorid als Einsatzprodukt für kontinuierliche Verfahren besonders geeignet.

Nachteilig ist allein der an sich sehr hohe Dampfdruck, den Cyanurchlorid schon an seinem Schmelzpunkt besitzt. Durch ihn ist die Konzentration des Cyanurchlorids in gasförmiger Form in dem Raum oberhalb seiner Schmelze stets verhältnismäßig groß.

Dadurch können aber Schwierigkeiten auftreten, wenn in dem Verfahrensfluß Stellen mit einer Temperatur entstehen, die bei oder unterhalb des Desublimationspunktes von Cyanurchlorid liegen.

Dies ist z. B. der Fall, wenn in die Schmelze kontinuierlich festes Cyanurchlorid zum Aufschmelzen eingeführt wird. Die Einführung wird immer in den Gasraum vorgenommen.

Da das feste Cyanurchlorid selbstverständlich eine tiefere Temperatur als der Gasraum besitzt, tritt an der Einführungsstelle für das feste Cyanurchlorid ein Temperaturabfall ein, der eine Krustenbildung hervorruft.

Übliche gasabschließende Einführungsorgane für feste Stoffe, z. B. Zellradschleusen, unterlagen sämtlich einem Zuwachsprozeß, der zu häufigeren Unterbrechungen, vor allem in kontinuierlichen Verfahren, unter Verwendung von flüssigem Cyanurchlorid führte.

Zweck der Anmeldung ist ein Verfahren zur einfacheren Handhabung bei der Herstellung von flüssigem Cyanurchlorid, vorzugsweise in kontinuierlichen Verfahren.

Es wurde nun gefunden, daß sich festes Cyanurchlorid in den Dampfraum oberhalb einer Cyanurchloridschmelze einführen läßt, wenn man das Cyanurchlorid mit Hilfe einer Förderschnecke, welche die Ausbildung eines gasdichten Stopfens von Cyanurchlorid in ihrem letzten Rohrabschnitt bewirkt, überführt.

Die Konstruktion der Schnecke muß in dem Produkt-Einfüllbereich derartig gestaltet sein, daß der Füllgrad so hoch wie möglich ist.

Das ist z. B. bei einer Schnecke der Fall, deren Einfüllbereich für das Cyanurchlorid Schneckengänge mit einer geringeren Steigung besitzt als im übrigen Schneckenrohr. Unter dem Ausdruck »im Einfüllbereich« wird nicht nur der Bereich direkt unter der Einfüllöffnung verstanden, sondern — wenn es zur Durchführung der Erfindung nötig sein sollte — werden auch darunter die ersten Schneckengänge im Anschluß an die Einfüllstelle verstanden.

Durch die Verwendung verschieden großer Steigungen der Schneckengänge ist es nämlich möglich, das Cyanurchlorid im Schneckenteil der Förderschnecke so zu verdichten, daß sich im letzten Rohrabschnitt vor Eintritt in den Gasraum ein gasdichter Pfropfen aus Cyanurchlorid bildet.

Dieser Pfropfen ist in der Lage, die Schnecke gegen den Gasraum über der Schmelze völlig abzuschließen, so daß die obengenannten Verkrustungen nicht mehr auftreten können und ein reibungsloses Einführen des festen Cyanurchlorids in die Schmelze möglich ist. Dies ist besonders wesentlich bei kontinuierlicher Durchführung des Verfahrens. Hier bildet sich der Pfropfen bei fortlaufender Zudosierung des festen Cyanurchlorids immer gasdicht nach, auch wenn sein äußeres Ende nach Erreichen des Gasraumes automatisch abgebrochen ist.

Besonders geeignet ist eine Schnecke, bei der der letzte Rohrabschnitt vor Eintritt in den Gasraum keine Wendeln enthält.

Jedoch haben sich auch Schnecken als günstig erwiesen, bei denen am Rohrende eine gewichtsbelastete Klappe angebracht ist. Dies kann auch der Fall sein bei einer Schnecke, deren letzter Rohrabschnitt wendelfrei ist.

Die Größe der Steigungen in den Schneckengängen, sowie die Länge der einzelnen zu den verschiedenen Schneckengängen gehörenden Rohrabschnitte werden durch Vorversuche, die die Bedingungen zur Bildung des gasdichten Pfropfens zum Ziel haben, bestimmt. Zu diesen Bedingungen gehört auch die Länge des letzten Rohrabschnittes, in dem der Pfropfen gebildet wird.

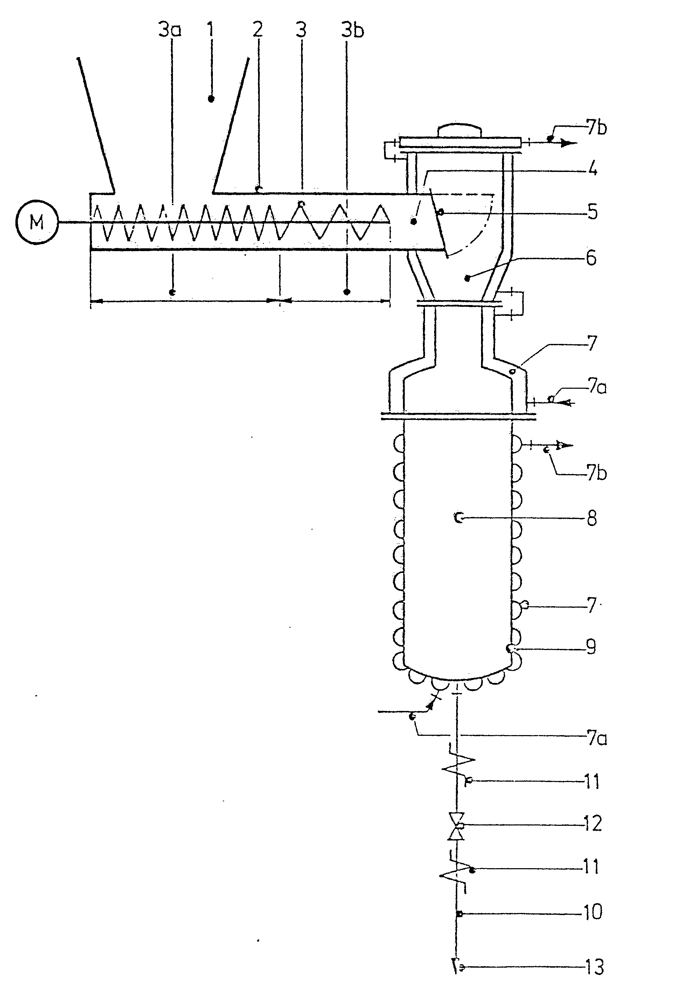

Abb. 1 betrifft beispielhaft eine Anordnung, bei der eine Schnecke mit einem wendelfreien Rohrabschnitt oder einer Klappe dargestellt ist, sowie zwei verschiedenen Steigungen der Schneckengänge.

Durch die Füllvorrichtung 1 fallen die festen Cyanurchloridpartikel — im allgemeinen in handelsüblicher Größe — auf die Förderschnecke 3, die sich in dem Förderrohr 2 befindet.

Der Steigungsbereich 3a mit der kleineren Steigung von z. B. 50 mm befindet sich im Einfüllbereich der Schnecke, der Steigungsbereich 3b mit einer Steigung von z. B. 80 mm im Rohrabschnitt hinter dem Einfüllbereich.

Nach Passieren der Abschnitte 3a und 3b gelangt das verdichtete Cyanurchlorid in den wendelfreien Rohrabschnitt 4, der entweder durch die Klappe 5 ersetzt werden kann — die sich in diesem Fall an den Steigungsbereich 3b anschließt — oder aber mit der Klappe 5 noch zusätzlich versehen wird, siehe Abb. 1.

Sowohl in dem wendelfreien Teil 4 wie durch Anschließen der Klappe 5 an den Bereich 3b bildet sich der gasdichte Pfropfen (nicht gezeigt). Bevorzugt ist eine Förderschnecke mit wendelfreiem Rohrabschnitt.

Angetrieben wird die Schnecke durch den Motor M.

Das Schneckenrohr 2 ragt mit seinem letzten Rohrabschnitt in den Behälter 9, der in den

Schmelzraum 8 und den Gasraum 6 unterteilt ist. Der Schmelzbehälter 9 ist von einem Heizmantel 7 umgeben, in den bei 7a das Heizmedium eintritt und bei 7b den Mantel wieder verläßt.

Als Heizmedium dienen alle für den notwendigen Temperaturbereich bekannten Stoffe, wie Dampf oder Wärmeträgeröle.

Über Leitung 10 mit dem Ventil 12 und der Leitungsbeheizung 11 wird die Schmelze abgenommen.

Zwar sind abdichtende Fördervorrichtungen, die mit Materialstopfen aus dem zu fördernden Material arbeiten, an sich bekannt, siehe DE-A-2 165 855 und DE-B-1 127 334, ebenso Förderanordnungen mit Förderschnecken, siehe US-A-3 460 869. Die in diesen Patentschriften genannten Materialien sind jedoch keine leicht schmelzenden und sublimierbaren Stoffe, wie Cyanurchlorid, sondern durch die dort angegebenen Materialstopfen soll nur erreicht werden, daß feste Stoffe aus einem Raum, in dem ein niedriger Druck herrscht, in einen Raum mit höherem Druck gefördert werden können, ohne daß Strömungsumkehr eintritt.

Bei der direkten Förderung von festem Cyanurchlorid in den Gasraum oberhalb einer Cyanurchloridschmelze setzt dagegen — wie gesagt — bei Berührung des heißen Gases, das Cyanurchloriddampf enthält, mit dem kalten, festen Cyanurchlorid sofort eine Desublimation ein, verbunden mit einem Anschmelzen des einzuführenden Cyanurchlorids. Hierdurch werden die fördernden Teile schließlich durch Krustenbildung verstopft, und eine weitere Einführung von Cyanurchlorid ist unmöglich.

Durch das erfindungsgemäße Verfahren ist es nun durch Ausbildung eines Stopfens von verdichtetem Cyanurchlorid, der den Dampfraum oberhalb der Cyanurchloridschmelze gasdicht gegenüber den kälteren Förderteilen abdichtet, möglich geworden, ohne Schwierigkeiten festes Cyanurchlorid in den Schmelzbehälter zu fördern.

Die Erfindung wird durch die folgenden Beispiele weiter erläutert:

Beispiel 1

In einer Anordnung nach Abb. 1 wurden in Intervallen 5 × 50 kg Cyanurchlorid innerhalb von je 8 Minuten durch eine Schnecke 3 in einen Aufschmelzbehälter 9 gefördert. Die Länge der Schnecke 3 betrug 1000 mm und der Durchmesser 180 mm. Die Wendeln endeten 200 mm vor dem Trogausgang. Die Steighöhe in Steigungsabschnitt 3a betrug 50 mm, die in Steigungsabschnitt 3b 80 mm. Eine Klappe 5 war nicht vorgesehen. Das Förderrohr 2 war unbeheizt. Das feste Cyanurchlorid hatte Raumtemperatur. Die Temperatur der Schmelze im Aufschmelzbehälter 9 wurde auf 170° C gehalten (Ölbeheizung), der den Dampfraum 6 umgebende Heizmantel 7 wurde durch aufgelegte Kupferrohre mit 10 bar Dampf beheizt. Entlüftet wurde die Apparatur in einem Laugewäscher (nicht gezeigt).

Beim Abstellen der Schnecke 3 wirkte der am Schneckenausgang befindliche Produktstopfen als Dampfsperre für das in den Schneckengängen verbleibende Produkt, so daß ein weiteres Trennorgan zwischen fester- und Dampfphase nicht nötig war. Der feste Pfropfen aus Cyanurchloridschmelze ließ sich beim Wiederanfahren sowie bei Förderung von frischem Produkt durch die Schnecke 3 jeweils mühelos wieder aufbrechen und wegdrücken.

Beispiel 2

In einer Anordnung wie in Beispiel 1 wurden analog, aber kontinuierlich, 500 kg Cyanurchlorid in vorgelegte Schmelze innerhalb von 60 Minuten eingetragen und am Bodenauslauf 10 des Aufschmelzbehälters 9 an Punkt 13 kontinuierlich entnommen. Danach wurde die Schnecke 3 abgestellt und die Heizung erst des Aufschmelzbehälters 9, dann des Gasraumes 6 abgestellt. Die Gänge der Schnecke waren danach — soweit sichtbar — absolut belagfrei. Im Förderrohr 2 saß nahe dem Ausgang ein fester Produktstopfen, der aber später durch die Schnecke 3 ohne Schwierigkeiten aufgedrückt werden konnte. Die Wandungen des Gasraumes 6, sowie der vordere, in den Gasraum 6 hineinragende Teil des Förderrohres 2 waren vollkommen frei von Belägen.

Patentansprüche

1. Verfahren zum Einführen von festem Cyanurchlorid in den Dampfraum oberhalb einer Cyanurchloridschmelze, dadurch gekennzeichnet, daß man das Cyanurchlorid mit Hilfe einer Förderschnecke, welche die Ausbildung eines gasdichten Stopfens von Cyanurchlorid in ihrem letzten Rohrabschnitt bewirkt, überführt.

2. Verfahren nach Anspruch 1, dadurch gekennzeichnet, daß man das Cyanurchlorid mit Hilfe einer Förderschnecke überführt, in derem letzten Rohrabschnitt kein Schneckengewinde vorhanden ist.

3. Verfahren nach Anspruch 1 oder 2, dadurch gekennzeichnet, daß man das Cyanurchlorid mit Hilfe einer Förderschnecke überführt, an deren Ende eine gewichtsbelastete Klappe vorhanden ist.

Claims

1. A process for the introduction of solid cyanuric chloride into the steam chamber above a cyanuric chloride melt, characterized in that the cyanuric chloride is transported by means of a screw conveyor which causes the formation of a gas-tight plug of cyanuric chloride in its last tubular section.

2. A process according to claim 1, characterized in that the cyanuric chlorid is transported by a screw conveyor, the last tubular section of which does not have a screw thread.

3. A process according to claim 1 or 2, characterized in that the cyanuric chloride is transported by a screw conveyor, the end of which is provided with a weight-loaded flap.

## Revendications

1. Procédé d'introduction de chlorure de cyanuryle solide dans le compartiment à vapeur situé au-dessus d'une masse fondue de chlorure de cyanuryle, procédé caractérisé en ce que l'on transfère le chlorure de cyanuryle à l'aide d'une vis transporteuse, qui permet la formation d'un bouchon étanche aux gaz en chlorure de cyanuryle, dans sa dernière section tubulaire.

2. Procédé selon la revendication 1, caractérisé en ce que l'on transfère le chlorure de cyanuryle à l'aide d'une vis transporteuse dans laquelle la dernière section tubulaire ne comporte pas de spires.

3. Procédé selon l'une des revendications 1 et 2, caractérisé en ce que l'on transfère le chlorure de cyanuryle à l'aide d'une vis transporteuse, à l'extrémité de laquelle est prévu un clapet lesté.